# EUROPEAN PATENT APPLICATION

(11) **EP 1 719 543 A1**
(43) Date of publication of application: **08.11.2006**
(21) Application number: 05009830.0
(22) Date of filing: 04.05.2005
(51) Int. Cl.: A61P 1/08, A61P 1/12, A61K 31/45, A61K 38/07, A61K 31/19, A61K 45/00, A61K 31/192, A61K 31/16, A61K 31/20, A61K 31/165

(54) **Use of histone deacetylase inhibitors for the treatment of gastrointestinal distress**

(71) Applicant: Asan Labs., Ltd., 106 Taipei City (TW)
(72) Inventor: Chung, Yih-Lin, Daan Chiu, Taipei (TW); Pui, Nam-Mew, Da-an District, Taipei City 106 (TW)
(74) Representative: Bockhorni, Josef

(57) **Abstract**

Use of a pharmaceutical composition for the manufacture of a medicament for treating and/or preventing acute and chronic gastrointestinal distress including nausea, vomiting, lactose intolerance, obstructive symptoms, diarrhea, mucositis, bleeding, weight loss, and malnutrition in a subject who is immunocompromised or receives a planned course of chemotherapy and/or radiotherapy. The pharmaceutical composition comprises a histone deacetylase inhibitor optionally in conjunction with a second agent. It is further provided use of a histone deacetylase inhibitor for the manufacture of a medicament for treating and/or preventing cachexia, cancer-related fatigue, or chronic fatigue syndrome.

## Description

### BACKGROUND

The invention relates to the use of inhibitors of histone deacetylase (HDAC) to treat or prevent acute and chronic gastrointestinal (GI) distress including nausea, vomiting, lactose intolerance, obstructive symptoms, diarrhea, mucositis, bleeding, weight loss, and malnutrition induced by chemotherapy and radiotherapy.

### A. Chemotherapy and radiotherapy-induced acute GI distress such as nausea and vomiting

Chemotherapy and radiotherapy-induced nausea and vomiting causes a significant deterioration in the quality of life as well as physical and cognitive functioning, resulting in delay or interruption of potentially curative therapy.

In fact, chemotherapy and radiotherapy-induced nausea and vomiting can be so severe that the patient refuses further treatment. Five types of nausea and vomiting are associated with the use of chemotherapeutic agents and/or radiation: (1) acute chemotherapy and radiotherapy-induced nausea and vomiting, which occurs within the first 24 hours of treatment; (2) delayed chemotherapy and radiotherapy-induced nausea and vomiting, which occurs 24 hours or more after treatment; (3) anticipatory chemotherapy and radiotherapy-induced nausea and vomiting, which begins prior to treatment; (4) breakthrough chemotherapy and radiotherapy-induced nausea and vomiting, which occurs despite patients begin treated with preventive therapy; (5) refractory chemotherapy and radiotherapy-induced nausea and vomiting, which occurs during subsequent cycles of treatment when antiemetic prophylaxis or rescue therapy has failed in earlier cycles.

The mechanisms of chemotherapy and radiotherapy-induced nausea and vomiting are not well defined, but evidences suggest that chemotherapy and radiotherapy-induced nausea and vomiting, in part, by causing enterochromaffin cells lining the GI tract mucosa in response to cell damage (mucositis) to release serotonin and other neuroactive agents to bind to their receptors in afferent vagal nerves in the GI tract and send impulses to the vomiting center (VC) and the chemoreceptor trigger zone (CTZ) in the parvicellular reticular formation in the lateral medullary region of the brain stem and the area postrema near the 4th ventricle of central nervous system (CNS), respectively (Navari RM. J. Supp. Oncol. 1:89-92, 2003; Grunberg SM. J. Supp. Oncol. 2:1-12, 2004). Activation of the CTZ also triggers the release of neurotransmitters that further activate the VC. The CTZ neurotransmitters considered related to chemotherapy and radiotherapy-induced nausea and vomiting include, but are not limited to, dopamine, serotonin, histamine and norepinephrine. Direct links exist between the higher CNS centers and the VC/CTZ. The efferent branches of cranial nerves V, VII and IX, as well as the vagus nerve and sympathetic trunk then produce the complex coordinated set of muscular contractions, cardiovascular responses and reverse peristalsis that characterize vomiting.

There are a number of agents that are clinically used for the treatment of chemotherapy and radiotherapy-induced nausea and vomiting. These agents include: anticholinergics, antihistamines, phenothiazines, butyrophenones, cannabinoids, benzamides, glucocorticoids, benzodiazepines, 5-HT₃receptor antagonists and tricyclic antidepressants. It is, however, still a need to improve treatment regimens.

For example, extrapyramidal symptoms, such as, dystonia and akathisia, sedation, anticholinergic effect and orthostatic hypotension make the use of the phenothiazines a less than desirable therapy. Drowsiness is a significant side effect of anticholinergics; Sedation and anticholinergic effects the major drawbacks of antihistamines. Side effects of butyrophenones include akathisia, dystonia and hypotension. Cannabinoids have shown limited efficacy and side effects of euphoria, dizziness, paranoid ideation and somnolence. Side effects of the benzodiazapines include perceptual disturbances, urinary incontinence, hypotension, diarrhea, sedation and amnesia. Steroids have shown little efficacy as a single agent and side effects of hyperglycemia, euphoria, insomnia and rectal pain. The undesirable side effects of the anticholinergic properties of the tricyclic antidepressants include dry mouth, constipation, blurred vision, urinary retention, weight gain, hypertension, palpitations and arrhythmia. The use of 5-HT₃ receptor antagonists such as ondansetron, granisetron and tropisetron has been shown to be less effective for delayed nausea and vomiting than for acute symptoms. Efficacy of the 5-HT₃ receptor antagonists appears to be less pronounced for moderate emetogenic chemotherapy regimens than for cisplatin-containing regimens. Control of the 5-HT₃ receptor antagonists over nausea appears to be significantly less than control over vomiting. Further, the efficacy of the 5-HT₃ receptor antagonists appears to diminish across repeated days and across repeated chemotherapy cycles (Morrow et al., Cancer 76:343-357, 1995).

As such, improved methods for the prevention and treatment of nausea and vomiting are needed.

When GI tract is exposed to chemotherapy and/or radiotherapy, in response to cell damage (mucositis), the enterochromaffin cells lining the GI tract release neurotransmitters to relay signals to the VC/CTZ of CNS, resulting in chemotherapy and radiotherapy-induced nausea and vomiting. However, only to block the actions of neurotransmitters in the GI tract and CNS has been shown to be ineffective to treat all types of chemotherapy and radiotherapy-induced nausea and vomiting. Thus, in addition to target the active neurotransmitters and their receptors in the GI tract and the CNS, an approach or agent that can prevent the cell damage (mucositis) to maintain the integrity of epithelium of GI tract to decrease the release of neurotransmitters from the GI tract to the vomiting centers of CNS may be useful in preventing and treating chemotherapy and radiotherapy-induced nausea and vomiting.

### B. Chemotherapy and radiation-induced mucositis

Oral mucositis and GI mucositis have been considered to be elements of alimentary mucositis, with regional differences being due to the specialized needs of each area (Keefe, DMK. Supportive Care Cancer. 12:6-9, 2004). It has become clear that instead of mucositis simply arising from the direct effects of chemotherapy and/or radiotherapy on basal epithelial stem cells, mucositis appears to be the consequence of a sequence or series of biological events that begin in the connective tissue (endothelial and mesenchymal cells) of submucosa and target the epithelial cells (Sonis ST et al., J. Supp. Oncol. 2:21-31, 2004). The pathogenesis of mucositis induced by chemotherapy and/or radiation can be thought of as occurring in five phases:
Phase I - Initiation. Initiation of chemotherapy and radiation-induced cell damage is characterized by generation of reactive oxygen species (ROS) to break double-strand DNA, and coincidence of activation of ROS-independent signal pathways, such as protein kinase c (PKC).
Phase II - Damage Message Generation. Damage message generation is characterized by activation of transcriptional factors such as NF-κB to turn on pro-inflammatory cytokine expression, such as TNF-α, IL-1β, and IL-6.
Phase III - Damage Signal Amplification. Damage signal amplification by positive feedback loops between NF-κB and TNF-α further increases the numbers and levels of pro-inflammatory cytokines; TNF-α not only further increases the activity of NF-κB but also induces the extrinsic apoptotic pathway, resulting in epithelial cell death.
Phase IV - Ulceration and Infection. Ulceration and infection (moderate to severe mucositis) are characterized by primary loss of epithelial cells and secondary colonization of bacteria (causing pain, inflammation and loss of function) ;
Phase V - Healing. The healing process of re-epithelium is stimulated by signals from the exposed extracellular matrix and growth factors secreted from the fibroblasts in the submucosa.

Although there might be some mucosal erythema during phase I to III, tissue integrity is still in place and patients have few symptoms until ulcerative mucositis develops due to epithelial cell death in phase IV. The process of tissue injury from initiation (phase I) to healing (phase V) is believed to recur at different sites on the mucosa of the GI tract following each fraction of radiotherapy or each cycle of chemotherapy throughout the whole treatment course. Thus, each of the five phases previously described offers potential targets for the prevention, amelioration, and/or acceleration of healing of cell damage or mucositis induced by chemotherapy and/or radiation. However, although the mechanisms that underlie the aetiology have provided a range of therapeutic targets, a key challenge to the development of any therapy that is aimed at modulating radiation- or chemotherapy-associated toxicity is to ensure that it targets normal tissue effectively, but does not diminish the tumoricidal impact of the antineoplastic treatment by radiotherapy and chemotherapy.

In order to prevent the appearance of ulcerative mucositis, it might be better to stop the pathogenesis before phase IV. However, if phase I is blocked, the tumoricidal effects of radiation and chemotherapy might be compromised because the generation of ROS to break double strand DNA is the major mechanism for tumor killing by radiation and chemotherapy. Therefore, it seems that only phase II (such as NF-κB) and phase III (such as TNF-α) are better targets for prevention of mucositis without compromising tumor control.

After chemotherapy and radiation injury, the production of cytokines such as TNF-α and growth factors such as TGF-β in irradiated tissues perpetuates and augments the inflammatory response, while promoting fibroblast recruitment and proliferation but inhibiting epithelial cell growth (Hill, RP. , et al., Int. J. Radiat. Oncol. Biol. Phys., 49: 353-365, 2001). The amplified injury response to chemotherapy and radiation by the persistent secretion of TNF-α and TGF-β from epithelial, endothelial, and connective tissue cells, which is possibly caused by a modification in the genetic programming of cell differentiation and proliferation, leads to the histological modifications that characterize mucositis (Zhou, D., et al., Int. J. Radiat. Biol., 77: 763-772, 2001). Thus, the chemotherapy and radiation-induced cell injury could be regarded as a genetic disorder in the wound healing process.

### C. Histone deacetylase (HDAC) inhibitor as a gene modulator

HDAC inhibitors as a class of compounds with abilities in multiple gene regulation can modulate the expression of a specific set of genes by increasing histone acetylation, thereby regulating chromatin structure and accessibility of target genes for transcription and thus treating diseases (Marks, PA., et al., J. Natl. Cancer Inst., 92: 1210-6, 2000). HDAC inhibitors act selectively on gene expression, altering the expression of only about 2% of the genes expressed in cultured tumor cells. By modulating specific genes related to cell cycle inhibitors, tumor suppressors and oncogenes, HDAC inhibitors have shown to be potent inducers of growth arrest, differentiation, and/or apoptotic cell death of transformed cells *in vitro* and *in vivo.* The effects of HDAC inhibitors induce bulk histone acetylation, resulting in apoptotic cell death, terminal differentiation, and growth arrest in tumor cells, but no toxicity in normal cells (Richon, VM., et al., Proc. Natl. Acad. Sci. USA., 97: 10014-10019, 2000; Van Lint, C., et al., Gene Expr., 5: 245-243, 1996). In addition, the modulation of chromatin conformation by HDAC inhibitors can further radiosensitize tumors whose cells are intrinsically radioresistant (Ferrandina, G., et al., Oncol. Res., 12: 429-440, 2001; Miller, AC., et al., Int. J. Radiat. Biol., 72: 211-218, 1997; Biade, S., et al., Int. J. Radiat. Biol., 77: 1033-1042, 2001). The epigenetic modification of chromatin structure suggests that HDAC inhibitors could be therapeutic candidates not only for cancers but also for genetic disorders (Jaenisch, R., et al., Nat. Genet., 33: 245-254, 2003; Garber, K., et al, J. Natl. Cancer Inst., 94: 793-795, 2002). On the other hand, HDAC inhibitors can also induce non-histone protein hyperacetylation. The hyperacetylation of nonhistone proteins such as ribosomal S3 or the Rel-A subunit of NF-κB inhibits the NF-κB activity and suppresses the pro-inflammatory cytokine production (TNF-α, Il-1β, I1-6, IL-8, TGF-β) (Chen, L., et al., Science, 293: 1653-1657, 2001). HDAC inhibitors have demonstrated the anti-inflammatory effects in many inflammation diseases such as ulcerative colitis and autoimmune diseases (Segain, JP., et al., Gut, 47: 397-403, 2000; Mishra, N., et al., Proc. Natl. Acad. Sci. USA., 98: 2628-2633, 2001; Leoni, F., et al. , Proc. Natl. Acad. Sci. USA, 99: 2995-3000, 2002; Chung, YL., et al., Mol. Ther. 8: 707-717, 2003).

Our previous study (Chung, YL, et al., Mol. Cancer Ther. 3: 317-325, 2004) demonstrated that in addition to suppressing tumor growth, HDAC inhibitors are also effective at preventing and treating radiation-induced dermatitis and promoting wound healing by downregulating the expression of TNF-α and TGF-β.

A study (Reddy P, et al., Proc. Natl. Acad. Sci. USA. 101:3921-6, 2004) also revealed that the HDAC inhibitor as an antitumor agent reduces the production of pro-inflammatory cytokines such as TNF-α to prevent acute graft-versus-host disease after bone marrow transplantation.

HDAC inhibitors have been found to be important for proper intestinal epithelial cell regulation by suppression of NF-κB activation and IL-8 production, and be an effective treatment for ulcerative colitis (Yin L, et al., J. Biol. Chem. 276:44641-6, 2001; Huang N, et al., Cytokine 9:27-36, 1997) .

### SUMMARY

Accordingly, there is provided a composition and method for treating or preventing acute and chronic GI distress including nausea, vomiting, lactose intolerance, obstructive symptoms, diarrhea, mucositis, bleeding, weight loss, and malnutrition in a subject who is immunocompromised or receives a planned course of chemotherapy and/or radiation therapy. The method comprises administering to the oral, pharyngeal, esophageal, or gastrointestinal tissues of the subject a therapeutically effective amount of an HDAC inhibitor alone or in combination with a second agent and a pharmaceutically acceptable carrier or a pharmaceutically acceptable salt thereof to treat or prevent cell damage, maintain integrity of epithelium of GI tract and decrease afferent vagal inputs from the GI tract to the vomiting center of central nervous system (CNS). It is also provided a composition and method for protection of normal tissues from chemotherapy and/or radiotherapy-induced injuries without the risk of tumor protection in cancer therapy. A pharmaceutical composition and method to treat or prevent cachexia, cancer-related fatigue or chronic fatigue syndrome are further provided because cytokine release, GI distress and tumor growth could be suppressed by using the HDAC inhibitors.

The HDAC inhibitors have been shown to upregulate tumor suppressors, downregulate oncogenes and repress pro-inflammatory cytokines for inhibiting tumor growth and inflammatory response. The compounds can be administered orally, intraperitoneally, intrathecally, intraarterially, intranasally, intraparenchymally, subcutaneously, intramuscularly, intravenously, dermally, intrarectally, and topically. The dosage amounts are based on the effective concentration observed *in vitro* and *in vivo* studies. The varied and efficacious utility of the compounds is further illustrated by the finds that they may also be administered concomitantly or in combination with a second HDAC inhibitor, a 5-hydroxytryptamine3 (5-HT₃) receptor antagonist, a dopamine receptor antagonist, a DOPA-5-HT₃ receptor antagonist, a neurokinin (NK)-1 receptor antagonist, an anti-histamine, an anticholinergics a non-steroid anti-inflammation drug, a steroid, a growth factor, a cytokine, an anti-oxidant agent, a tricyclic antidepressant, a sedative agent, cannabinoids, a vitamin, or an antibiotics.

Drugs that have been used in the management of chemotherapy and radiotherapy-induced GI distress include anticholinergics, antihistamines, phenothiazines, butyrophenones, cannabinoids, benzamides, glucocorticoids, benzodiazepines, 5-HT₃ receptor antagonists and tricyclic antidepressants. None of these are able to effectively suppress all types of chemotherapy and radiotherapy-induced GI distress. To find an agent to protect normal tissues without the risk of tumor protection in chemotherapy and radiotherapy has been a long-sought goal in cancer treatment. However, the use of HDAC inhibitors or in combination with above or other agents to prevent mucositis, maintain integrity of GI tract and decrease afferent vagal inputs from the GI tract to the VC/CTZ of CNS for treatment or prevention of chemotherapy and radiotherapy-induced acute and chronic GI distress has not theretofore been suggested or disclosed. The use of HDAC inhibitors for protection of normal tissues without the risk of tumor protection in chemotherapy and/or radiotherapy has also not theretofore been suggested or disclosed. Moreover, because cytokine release, GI distress and tumor growth could be suppressed by using the HDAC inhibitors, it is further provided a method for treating or preventing cachexia, cancer-related fatigue or chronic fatigue syndrome in a patient who receives a planned course of chemotherapy and/or radiotherapy.

### BRIEF DESCRIPTION OF THE DRAWINGS

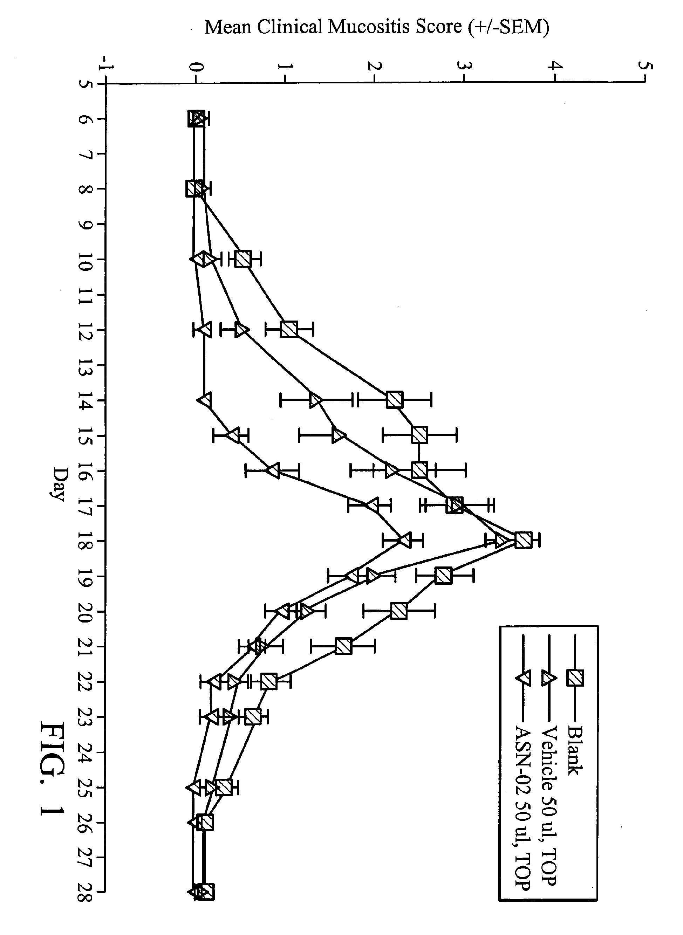

FIG. 1 is a diagram showing that the vehicle and blank control groups exhibit the expected clinical severe radiation-induced mucositis scores at the expected peak mucositis time at Day 14 to Day 18 following radiation, and the ASN-02 (phenylbutyrate) oral gel reduces the mean clinical mucositis scores relative to the vehicle and blank controls in incidence, severity and duration of radiation-induced mucositis.

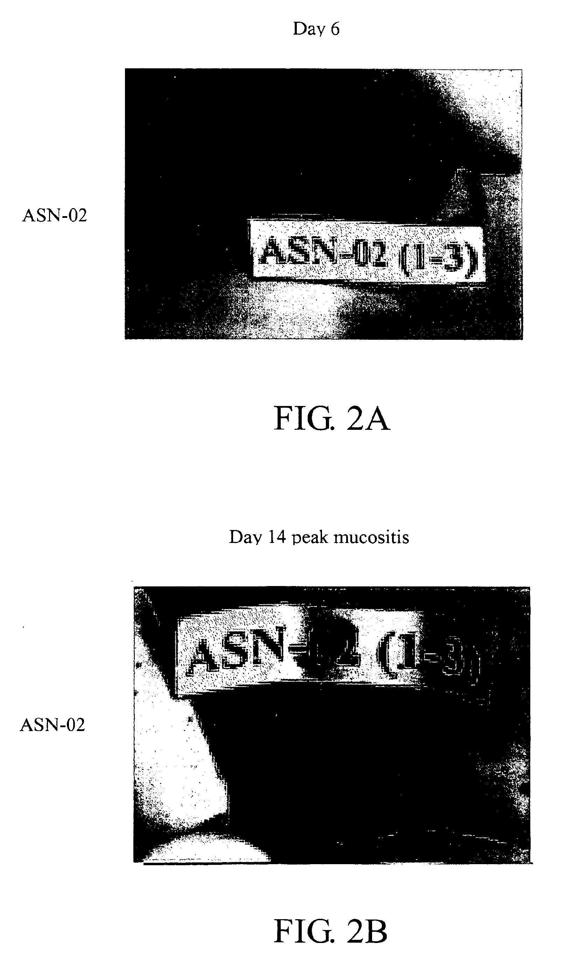

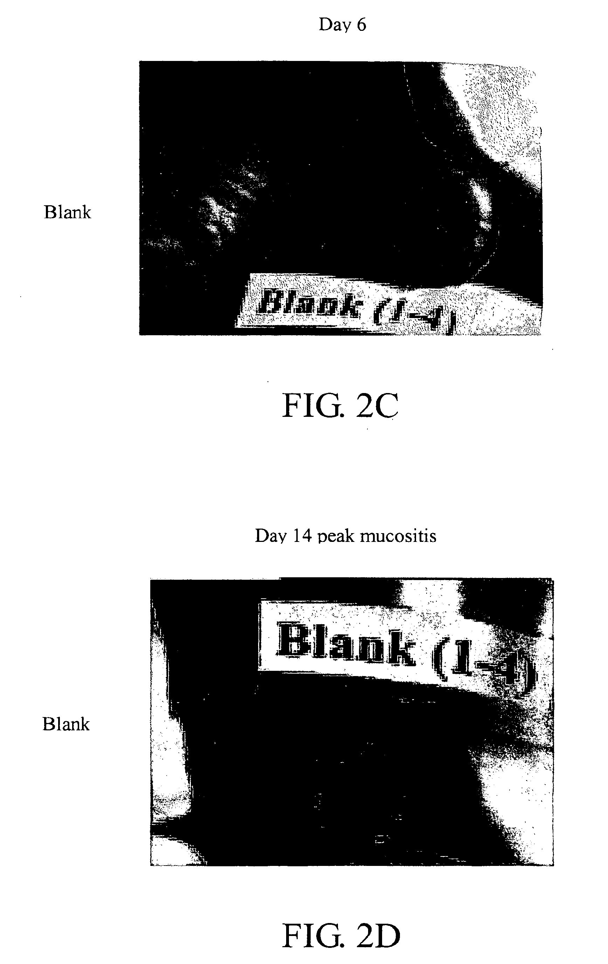

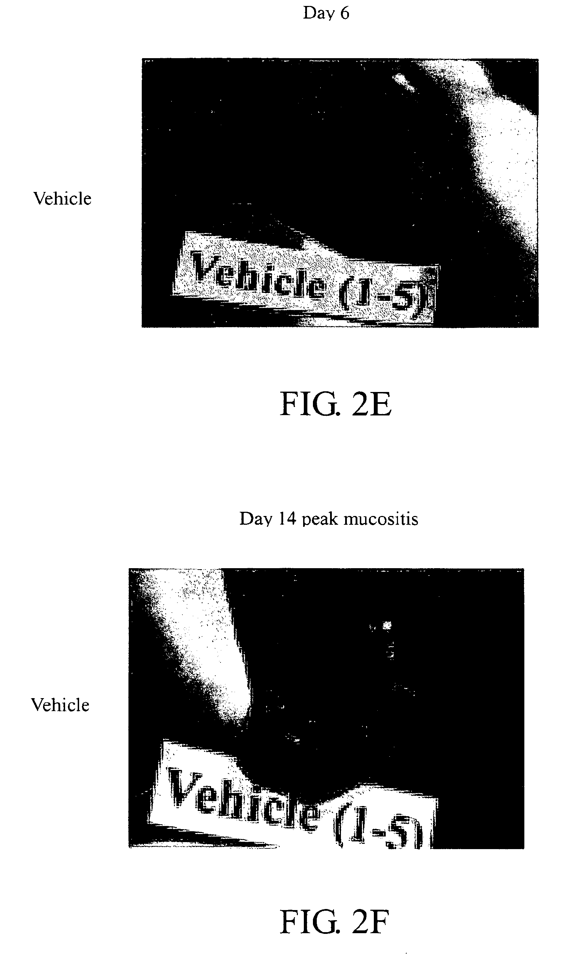

FIG. 2A to 2F are gross pictures showing that at Day 14 following radiation, the huge difference between the blank and vehicle control groups and the ASN-02 (phenylbutyrate)-treated group. Ulcerative mucositis appears at the blank and vehicle groups; in contrast, the buccal mucosa is still intact in the ASN-02 (phenylbutyrate)-treated group. FIG. 2A and 2B are the ASN-02-treated group; FIG. 2C and 2D the blank control group; FIG 2E and 2F the vehicle control group. FIG. 2A, 2C, and 2E are at Day 6; FIG. 2B, 2D, and 2F at Day 14.

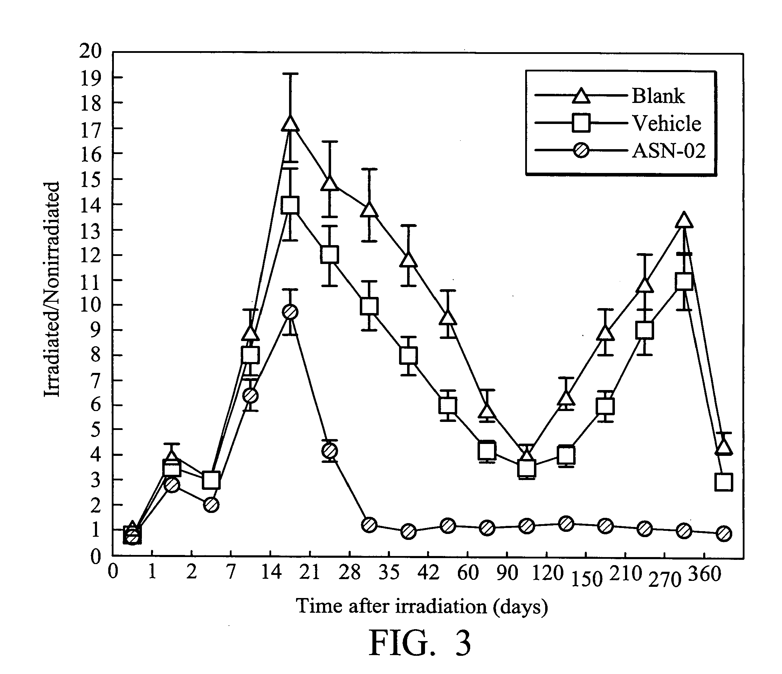

FIG. 3 is a diagram showing that temporal variation in mRNA levels of TNF-α in mucosa after irradiation, normalized to the internal control GAPDH and expressed as a ratio to levels in nonirradiated mucosa samples. Each point represents the mean of mRNA levels of 5 samples in the same group of blank, vehicle, or ASN-02 (phenylbutyrate). The timing of peak appearance of TNF-α upregulation correlated well with the development of mucositis. The HDAC inhibitor effectively suppressed the long-term aberrant expression of TNF-α when compared to the blank and vehicle control.

### DETAILED DESCRIPTION

A pharmaceutical composition and method for the prevention and treatment of acute and chronic GI distress including nausea, vomiting, lactose intolerance, obstructive symptoms, diarrhea, inucositis, bleeding, weight loss, and malnutrition in a subject in need thereof are provided. The subject is immunocompromised or receives a planned course of chemotherapy and/or radiation therapy. The radiation therapy is an ionizing radiation, an external beam radiation, a brachytherapy, a radiopharmaceutical agent, a radioactive conjugate agent, or a radiolabeled antibody. The method comprises administering to the oral, pharyngeal, esophageal, or gastrointestinal tissues of the subject a therapeutically effective amount of an HDAC inhibitor or in combination with a second agent. The pharmaceutical composition comprises a therapeutically effective amount of an HDAC inhibitor or in combination with a second agent for preventing cell damage, maintaining integrity of GI tract and decreasing afferent vagal inputs from the GI tract to the VC/CTZ of CNS, resulting in preventing and treating acute and chronic GI distress. It is also provided a pharmaceutical composition and method using HDAC inhibitors for protection of normal tissues from chemotherapy and radiotherapy-induced injuries without the risk of tumor protection in cancer treatment. Based on suppressing cytokine release, GI distress and tumor growth using the HDAC inhibitors, a method for treating or preventing cachexia, cancer-related fatigue or chronic fatigue syndrome is further provided.

Acute tissue injury caused by chemotherapeutic agents and radiation is believed to be related to oxidative damage from the formation of free radicals. Thus, the pathogenesis of chemotherapy and radiotherapy-induced acute GI distress is thought to involve directly with cell damage or GI mucositis to release active neurotransmitters causing increase of afferent vagal inputs from the GI tract to the VC/CTZ of CNS that induces nausea and vomiting. The degree or intensity and duration of acute GI distress is closely related to the chemotherapeutic agents, radiation source, cumulative dose, dose intensity, the volume of affected intestine, and patient characteristics, i.e., female gender, younger age, a history of motion sickness, and consumption of minimal amounts of alcohol.

On the other hand, chronic GI mucositis induced by chemotherapy and radiotherapy is a late complication of cancer therapy, most commonly for oral cavity, esophagus, stomach, pancreas, liver, bile ducts, rectal, prostate and pelvic malignancies. It is often progressive, and may lead to a variety of clinical consequences including lactose intolerance, obstructive symptoms, chronic diarrhea, GI bleeding, weight loss, and malnutrition, which depends upon the extent of the injury. It usually develops six or more months after therapy (mean approximately 5 years, range two months to as long as 30 years) (Waddell BE., et al., J. Am. Coll. Surg. 189 (6) : 611-624, 1999). This contrasts with the timing of acute GI mucositis (characterized by nausea, vomiting, watery diarrhea and abdominal pain), which develops during or shortly after therapy and resolves within two to six weeks.

The prominent histopathologic features of chronic mucositis induced by chemotherapy and radiotherapy are those of an occlusive vasculitis with diffuse collagen deposition and fibrosis (Hasleton PS.; et al., Histopathology 9 (5) : 517-534, 1985) . The arteriolar walls may show a hyaline ring-like thickening and large foams cells beneath the intima. Telangiectasias may be seen. The intestinal segments and their associated serosa appear grossly thickened. Mucosal ulceration, necrosis and perforation may develop as the disease progresses. Progressive fibrosis leads to stricturing with dilation of proximal segments. The physiologic consequences may include altered intestinal transit, reduced bile acid absorption, increased intestinal permeability, bacterial overgrowth and lactose malabsorption (Yeoh E.; et al., Am. J. Med. 95(4):397-406, 1993).

Thus, the resulting clinical manifestations in acute and chronic GI distress induced by chemotherapy and radiotherapy may include nausea, vomiting, lactose intolerance, obstructive symptoms, diarrhea, weight loss, malnutrition, and bleeding.

Previous studies have shown how the late complications develop from the initial mucosal injury (Dorr W.; et al., Radiothe.r Oncol. 61(3):223-231, 2001). After chemotherapy and/or radiotherapy induces tissue injuries such as mucositis, the release of pro-inflammatory cytokines (TNF-α and TGF-β) in affected tissues perpetuates and augments the inflammatory response, while promoting fibroblast recruitment and proliferation but inhibiting epithelial cell growth. Especially, the amplified injury is responded by the persistent secretion of TNF-α and TGF-β from epithelial, endothelial, and connective tissue cells, which is possibly caused by a modification in the genetic programming of cell differentiation and proliferation. The chronic activation of TGF-β pathway also stimulates late tumorigenesis. Thus, the chemotherapy and radiotherapy-induced cell damage or mucositis resulting from aberrant gene overexpression in cytokines could be regarded as a genetic disorder that will lead to poor wound healing, progressive fibrosis and late tumorigenesis.

A class of gene modulators, HDAC inhibitors, activates and represses a subset of genes by remodeling the chromatin structure via the altered status in histone acetylation (Marks et al, J. Natl. Cancer Inst., 92: 1210-6, 2000; Kramer et al, Trends Endocrinol. Metab., 12: 294-300, 2001). Histone hyperacetylation results in the up-regulation of cell-cycle inhibitors (p21Cipl, p27Kipl, and p16INK4), the down-regulation of oncogenes (Myc and Bcl-2), the repression of inflammatory cytokines (interleukin (IL)-1, IL-8, TNF-α, and TGF-β), or no change (GAPDH and γ-actin) (Lagger et al, EMBO J., 21: 2672-81, 2002; Richon et al, Clin. Cancer Res., 8: 662-667, 2002; Richon et al, Proc. Natl. Acad. Sci. USA., 97: 10014-9, 2000; Van Lint et al, Gene Expr., 5: 245-3, 1996; Huang et al, Cytokine, 9: 27-36, 1997; Mishra et al, Proc. Natl. Acad. Sci. USA., 98: 2628-33, 2001; Stockhammer et al, J. Neurosurg., 83: 672-81, 1995; Segain et al, Gut, 47: 397-403, 2000; Leoni et al, Proc. Natl. Acad. Sci. USA, 99: 2995-3000, 2002). In addition to inducing histone hyperacetylation, HDAC inhibitors also induce hyperacetylation of nonhistone proteins such as ribosomal S3, p53 or the Rel-A subunit of NF-κB, modulate protein kinase C (PKC) activity, inhibit protein isoprenylation, decrease DNA methylation, and bind to nuclear receptors (Webb et al, J. Biol. Chem., 274: 14280-7, 1999; Chen et al, Science, 293: 1653-7, 2001). More and more different mechanisms also pointed to inhibition of NF-κB transcriptional activity after treatment with HDAC inhibitors. HDAC inhibitors have exhibited properties in inducing cell-cycle arrest, cell differentiation, and apoptotic cell death in tumor cells and in decreasing inflammation and fibrosis in inflammatory diseases (Warrell et al, J. Natl. Cancer Inst., 90: 1621-5, 1998; Vigushin et al, Clin. Cancer Res., 7: 971-6, 2001; Saunders et al, Cancer Res., 59: 399-404, 1999; Gottlicher et al, EMBO J., 20: 6969-78, 2001; Rombouts et al, Acta Gastroenterol. Belg., 64: 239-46, 2001). The effects of HDAC inhibitors induce bulk histone acetylation, resulting in apoptotic cell death, terminal differentiation, and growth arrest in tumor cells, however, these does not appear in normal cells because the sensitivity to apoptosis induction by HDAC inhibitors depends on the original state of cell differentiation as well as the acetylated histone status (Garber et al, J. Natl. Cancer Inst., 94: 793-5, 2002). In addition, the modulation of chromatin conformation by HDAC inhibitors can further radiosensitize tumors whose cells are intrinsically radioresistant, and also sensitize tumor cells to chemotherapy (Ferrandina et al, Oncol. Res., 12: 429-40, 2001; Miller et al, Int. J. Radiat. Biol., 72: 211-8, 1997; Biade et al, Int. J. Radiat. Biol., 77: 1033-42, 2001).

On the basis of the potential possibility in simultaneously, coordinately, selectively, and epigenetically manipulating the expression of tumor suppressors, oncogenes, and pro-inflammatory cytokines (TNF-α, IL-1, IL-6) and fibrogenic growth factors (TGF-β) by differentially remodeling the chromatins and inhibiting the NF-κB activity in normal and tumor cells, it is likely that HDAC inhibitors can exert their effects on the attenuation of cell damage or mucositis of GI tract by interfering with the phase II (NF-κB activation)/III (TNF-α production) of pathogenesis of chemotherapy and radiotherapy-induced mucositis (Sonis ST et al., J. Supp. Oncol. 2:21-31, 2004), and still exhibit anti-tumor effects and tumor radiosensitization in cancer treatment.

Inflammatory cytokines and neurotransmitters that mediate cell-to-cell communication are released in greater amounts in patients having cancers or receiving chemotherapy or radiotherapy. The cytokine release induced by cancers, chemotherapy or radiotherapy contributes to the development of fatigue by exerting effects on the endocrine system and neurotransmitters (Anisman H., et al., Can. Med. Assoc. J. 155:1075-1082, 1996), for example, as suggested in chronic fatigue syndrome (Moss RB., et al., J. Clin. Immunol. 19:314-316, 1999). High concentrations of TNF-α, IL-1, and IL-6 induced by cancers, chemotherapy or radiotherapy have been found to contribute to fever, weight loss, sweats, and anemias, as well as fatigue (Kurzrock R. Cancer 92:1684-1688, 2001; Wetzler M. et al. , Blood 84: 3142-3147, 1994) . Thus, the effects of the HDAC inhibitors in suppressing inflammatory cytokine release, preventing GI distress and inhibiting tumor growth can further decrease the development of cachexia, cancer-related fatigue or chronic fatigue syndrome.

Active compounds used to carry out the invention are, in general, histone hyperacetylating agents, such as HDAC inhibitors. Numerous such compounds are known. See, e.g., P. Dulski, Histone Deacetylase as Target for Antiprotozoal Agents, PCT Application WO 97/11366 (Mar.27, 1997). Examples of such compounds include, but are not limited to:
A. Trichostatin A and its analogues such as: trichostatin A (TSA); and trichostatin C (Koghe et al. 1998. Biochem. Pharmacol. 56:1359-1364) (Trichostatin B has been isolated but not shown to be an HDAC inhibitor).
B. Peptides, such as: oxamflatin [(2E)-5-[3-[(phenylsufonyl) aminophenyl]-pent-2-en-4-ynohydroxamic acid (Kim et al. Oncogene, 18:2461-2470 (1999)); trapoxin A (TPX)--cyclic tetrapeptide (cyclo-(L-phenylalanyl-L-phenylalanyl-D-pipecolinyl-L-2-a mino-8-oxo-9,10-epoxy-decanoyl)) (Kijima et al., J. Biol. Chem. 268, 22429-22435 (1993)); FR901228, depsipeptide (Nakajima et al., Ex. Cell Res. 241, 126-133 (1998)); FR225497, cyclic tetrapeptide (H. Mori et al . , PCT Application WO 00/08048 (Feb. 17, 2000)); apicidin, cyclic tetrapeptide [cyclo(N--O-methyl-L-tryptophanyl-L-isoleucinyl-D-pipecol inyl-L-2-amino-8-oxodecanoyl)] (Darkin-Rattray et al., Proc. Natl. Acad. Sci. USA 93, 13143-13147 (1996)); apicidin la, apicidin Ib, apicidin Ic, apicidin IIa, and apicidin IIb (P. Dulski et al. , PCT Application WO 97/11366) ; HC-toxin, cyclic tetrapeptide (Bosch et al., Plant Cell 7, 1941-1950 (1995)); WF27082, cyclic tetrapeptide (PCT Application WO 98/48825); and chlamydocin (Bosch et al., supra).
C. Hydroxamic Acid-Based Hybrid Polar Compounds (HPCs), such as: salicylihydroxamic acid (SBHA) (Andrews et al., International J. Parasitology 30, 761-8 (2000)); suberoylanilide hydroxamic acid (SAHA) (Richon et al., Proc. Natl. Acad. Sci. USA 95, 3003-7 (1998)); azelaic bishydroxamic acid (ABHA) (Andrews et al., supra); azelaic-1-hydroxamate-9-anilide (AAHA) (Qiu et al., Mol. Biol Cell 11, 2069-83 (2000)); M-carboxycinnamic acid bishydroxamide (CBHA) (Ricon et al., supra); 6-(3-chlorophenylureido)carpoic hydroxamic acid (3-Cl-UCHA) (Richon et al., supra); MW2796 (Andrews et al., supra); and MW2996 (Andrews et al., supra); pyroxamide, scriptaid, PXD-101, and LAQ-824. Note that analogs not effective as HDAC Inhibitors are: hexamethylene bisacetamide (HBMA) (Richon et al. 1998, PNAS, 95:3003-7); and diethyl bix(pentamethylene-N,N-dimethylcarboxamide)malonate (EMBA) (Richon et al. 1998, PNAS, 95:3003-7).
D. Fatty Acid compounds, such as: sodium butyrate (Cousens et al., J. Biol. Chem. 254, 1716-23 (1979)); isovalerate (McBain et al., Biochem. Pharm. 53:1357-68 (1997)); valproic acid; valerate (McBain et al., supra); 4-phenylbutyrate (4-PBA) (Lea and Tulsyan, Anticancer Research, 15, 879-3 (1995)); phenylbutyrate (PB) (Wang et al. , Cancer Research, 59, 2766-99 (1999)); propionate (McBain et al., supra); butrymide (Lea and Tulsyan, supra); isobutyramide (Lea and Tulsyan, supra); phenylacetate (Lea and Tulsyan, supra); 3-bromopropionate (Lea and Tulsyan, supra); tributyrin (Guan et al., Cancer Research, 60, 749-55 (2000)); arginine butyrate; isobutyramide; and valproate..
E. Benzamide derivatives, such as: MS-27-275 [N-(2-aminophenyl)-4-[N-(pyridin-3-yl-methoxycarbonyl) aminomethyl]benzamide] (Saito et al., Proc. Natl. Acad. Sci. USA 96, 4592-7 (1999)); and 3'-amino derivative of MS-27-275 (Saito et al., supra).
F. Other inhibitors, such as: depudecin [its analogues (mono-MTM-depudecin and depudecin-bisether) do not inhibit HDAC] (Kwon et al. 1998. PNAS 95:3356-61); and scriptaid (Su et al. 2000 Cancer Research, 60:3137-42).

Histone deacetylases (HDACs) used herein are enzymes which catalyze the removal of acetyl groups from lysine residues in the amino terminal tails of the nucleosomal core histones. As such, HDACs together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo.* HDACs can be divided into three classes based on structural homology. Class I HDACs (HDACs 1, 2, 3 and 8) bear similarity to the yeast RPD3 protein are located in the nucleus and are found in complexes associated with transcriptional co-repressors. Class II HDACs (HDACs 4, 5, 6, 7 and 9) are similar to the yeast HDA1 protein and have both nuclear and cytoplasmic subcellular localization. Both Class I and II HDACs are inhibited by hydroxamic acid-based HDAC inhibitors, such as SAHA. Class III HDACs form a structurally distant class of nicotinamide (NAD)-dependent enzymes that are related to the yeast SIR2 proteins and are not inhibited by hydroxamic acid-based HDAC inhibitors.

HDAC inhibitors used herein are compounds capable of inhibiting the deacetylation of histones *in vivo, in vitro* or both. As such, HDAC inhibitors inhibit the activity of at least one histone deacetylase. As a result of inhibiting the deacetylation of at least one histone, an increase in acetylated histone occurs, and accumulation of acetylated histone is a suitable biological marker for assessing the activity of HDAC inhibitors. Therefore, procedures which assay the accumulation of acetylated histones can be used to determine the HDAC inhibitory activity of compounds of interest. It is understood that compounds which inhibit histone deacetylase activity can also bind to other substrates and inhibit other biologically active molecules such as enzymes or non-histone proteins.

The HDAC inhibitor agents can be brought in the form of pharmaceutically acceptable salts. The pharmaceutically acceptable salts may be used so long as they do not adversely affect the desired pharmacological effects of the compounds. The selection and production can be performed by those skilled in the art. Examples of the pharmaceutically acceptable salts include alkali metal salts such as a sodium salt or a potassium salt, alkaline earth metal salts such as a calcium salt or a magnesium salt, salts with an organic base such as an ammonium salt, or a salt with an organic base such as a triethylamine salt or an ethanolamine salt.

The HDAC inhibitor agents may be administered orally or non-orally. In the case of oral administration, these agents may be administered in the form of soft and hard capsules, a tablet, a pill, a granule, a powder, a solution, a suspension, a mouthwash or the like. In the case of non-oral administration, these agents may be administered in the form of a cream, an ointment, a gel, a paste, a lotion, a patch, a suppository, a nanoparticle, a liposome formation, an injection solution, a drip infusion formulation, an enema or the like whereby continued membrane absorption can be maintained in the form of solid, viscous liquid, bioadhesive substance or suspension. The selection of the method for the preparation of these formulations and the vehicles or carriers, disintegrators or suspending agents, can be readily made by those skilled in the art. The HDAC inhibitor agents may contain a second agent and a pharmaceutically acceptable carrier or a pharmaceutically acceptable salt thereof.

The pharmaceutically acceptable carrier as used herein includes, but is not limited to, a biocompatible polymer having reverse-thermal gelation property, a polymer resin, a viscous polymer gel, a hydrogel, or a bioadhesive substance.

As recognized by those skilled in the art, the effective dosage varies depending on the route of administration, the excipient usage, and the possibility of co-use with other therapeutic treatments such as the use of a second agent such as a second HDAC inhibitor, a 5-hydroxytryptamine3 (5-HT₃) receptor antagonist, a dopamine receptor antagonist, a DOPA-5-HT₃ receptor antagonist, a neurokinin (NK)-1 receptor antagonist, an anti-histamine, an anticholinergics a non-steroid anti-inflammation drug, a steroid, a growth factor, a cytokine, an anti-oxidant agent, a tricyclic antidepressant, a sedative agent, cannabinoids, a vitamin, or an antibiotics.

The dopamine receptor antagonist can be phenothiazines, or butyrophenones. Examples of the 5-HT₃ receptor antagonist are dolasetron, granisetron, ondansetron, palonosetron, or tropistron. The DOPA-5-HT₃ receptor antagonist can be metoclopramide. Examples of the NK-1 receptor antagonist are vofopitant, CP-122,721, CJ-11,794, L-758,298, or aprepitant, In addition, the antibiotic includes, but are not limited to ganciclovir, acyclovir, famciclovir, or tetracycline. The growth factor can be keratinocyte growth factor (KGF), or granulocyte macrophage-colony stimulating factor (GM-CSF). The anti-oxidant can be amifostine, benzydamine, or N-acetylcysteine. The vitamin includes, but is not limited to, nicotinamide, vitamin B complex, vitamin C, or vitamin E.

Effective amounts and treatment regimens for any particular subject (e.g., human, dog, or cat) will also depend upon a variety of other factors, including the activity of the specific compound employed, age, body weight, general health status, sex, diet, time of administration, rate of excretion, severity and course of the disease, and the patient's disposition to the disease, but are usually from 0.001% to 100% by weight of the composition irrespective of the manner of administration. Active compounds may optionally be administered in conjunction with the second agent useful in preventing cell damage or mucositis, maintaining integrity of GI tract and decreasing afferent vagal inputs from the GI tract to the VC/CTZ of CNS in a subject who is immunocompromised or receives a planned course of chemotherapy and/or radiotherapy to prevent and treat acute and chronic GI distress including nausea, vomiting, lactose intolerance, obstructive symptoms, diarrhea, mucositis, bleeding, weight loss, and malnutrition. The second agent can be administered orally, intraperitoneally, intrathecally, intraarterially, intranasally, intraparenchymally, subcutaneously, intramuscularly, intravenously, dermally, intrarectally, or topically. In addition, the second agent can be formulated as a cream, a gel, an ointment, a paste, a mouthwash, a powder, a tablet, a pill, a granule, a capsule, a lotion, a suspension, a liposome formulation, a nanoparticle, a patch, a suppository, an enema, a drip infusion, or an injection solution. The second agent may optionally be administered concurrently or sequentially. As used herein, the word "concurrently" means sufficiently close in time to produce a combined effect (that is, concurrently may be simultaneously, or it may be two or more events occurring within a short time period before or after each other). As used herein, the administration of two or more compounds "concurrently" or "in combination" means that the two compounds are administered closely enough in time that the presence of one alters the biological effects of the other. The two compounds may be administered simultaneously or sequentially. Simultaneous administration may be carried out by mixing the compounds prior to administration, or by administering the compounds at the same point in time but at different anatomic sites or using different routes of administration.

In order that the invention described herein may be more readily understood, the following examples are set forth. It should be understood that the examples are for illustrative purpose only and are not to be construed as limiting this invention in any manner. All references cited herein are expressly incorporated by reference in their entirety.

### EXAMPLE

### Example 1: Composition Containing an HDAC Inhibitor for Treating and Preventing Chemotherapy and Radiotherapy-Induced Mucositis

An approach to selectively reduce mucosa morbidity without compromising the tumor-killing effects of chemotherapy and radiotherapy is a long-sought goal in cancer treatment. This example is to reveal a biologically based, topically applied regimen for treating mucositis. The vehicle acceptability and contact time of the medication in the mucosa are critical to the outcome of pharmacologic agents for treating mucositis. However, there are very few available vehicles or carriers for such an approach.

An HDAC inhibitor, phenylbutyrate, is formulated as an oral gel in a water-soluble resin with a food grade sweet flavoring. The backbone of the water-soluble resins is nonionic poly-ethylene oxide polymers for binding, lubricity, adhesion and emollient performance that meet all the specifications of the United States Pharmacopoeia-National Formulary. The water-soluble resins have applied to the usage such as controlled release solid dose matrix systems, transdermal drug delivery systems, and mucosal bio-adhesives. This feature allows the oral gel to undergo a phase transition, from a liquid upon oral intake, to a gel upon reaching body temperature in the body. This phase transition serves to increase contact time of the active compound phenylbutyrate with the mucosa, through the deposition of a thin coating on all contacted surfaces. An oral gel formulation can be selected because of its ease of use, broad coating of the mucosa, and patient acceptability/familiarity.

For example, each ml of the oral gel contains 50 mg sodium phenylbutyrate, 1.0% water soluble resins as bioadhesive substance, methylparaben as preservative, sodium saccharin as sweetener, a fragrant agent and purified water. This product is a transparent, colorless, and gel-like viscous solution with fragrance.

### Example 2: Treatment of Radiation-Induced Mucositis and Prevention of Malnutrition and Fatigue Using an HDAC Inhibitor

To evaluate the efficacy of the oral gel (ASN-02) containing 5% phenylbutyrate formulated in the water-soluble resins for the treatment of radiation-induced mucositis, a hamster animal model developed by Dr. Steve Sonis (Harvard School of Dental Medicine, Brigham and Women's Hospital, Boston, Mass.) was used.

Male Golden Syrian hamsters, 5 to 6 weeks of age, weighing approximately 90 g at study commencement were anesthetized with an intraperitoneal injection of sodium pentobarbital (80 mg/kg). The left buccal pouch was everted, fixed and isolated using a lead shield. Mucositis was induced using a standardized acute radiation protocol. A single dose of radiation (40 Gy/dose) was administered to the left buccal pouch mucosa of all animals on Day 0. Radiation was generated with a linear accelerator delivering a 6 MeV electron beam at a SSD of 100 cm at a rate of 300 cGy/minute. This radiation protocol produces "peak" oral mucositis at Day 14 to 18 after irradiation. Animals were dosed topically 3 times per day by applying 50 µl of ASN-02 or vehicle into the left (irradiated) buccal pouch per application from Day 1 to Day 28. Clinical mucositis, body weight, appetite, and activity were assessed every day starting on Day 6 to Day 28. Mucositis was evaluated by a visual scoring system. Following visual scoring, a photograph of each animal's mucosa was taken for comparison.

Description of clinical mucositis scoring: A score of 1-2 is considered to represent a mild stage of the mucositis, whereas a score of 3-5 is considered to indicate moderate to severe mucositis.

| Score | Description |
|---|---|
| 0 | Pouch completely healthy. No erythema or vasodilation. |
| 1 | Light to severe erythema and vasodilation. No erosion of mucosa. |
| 2 | Severe erythema and vasodilation. Erosion of superficial aspects of mucosa leaving denuded areas. Decreased stippling of mucosa. |
| 3 | Formation of off-white ulcers in one or more places. Ulcers may have a yellow/gray due to pseudomembrane. Cumulative size of ulcers should equal about 1/4 of the pouch. Severe erythema and vasodilation. |
| 4 | Cumulative size of ulcers should equal about 1/2 of the pouch. Loss of pliability. Severe erythema and vasodilation. |
| 5 | Virtually all of pouch is ulcerated. Loss of pliability (pouch can only partially be extracted from mouth. |

Data of visual scoring of mucositis and representative photographs showing the development of peak mucositis are shown in FIG. 1 and FIG. 2A-2F, respectively. Severity of mucositis increases with score. A score of 1-2 is considered to represent a mild stage of the mucositis, whereas a score of 3-5 is considered to indicate moderate to severe mucositis. Values are the mean clinical mucositis scores ± SEM per formulation treatment or control group (N=9 hamsters in the blank control group, N=9 hamsters in the vehicle group, and N=11 hamsters in the ASN-02 group). The vehicle and blank control groups exhibited the expected clinical mucositis score (i.e., a score of 3 to 4) at the expected peak mucositis time (i.e., 14 to 18 days post-irradiation) . Five animals of 11 in the ASN-02 group had mucositis scores between 2.0 and 3.0 at the peak (Day 18), and none in the ASN-02 group had score > 3.0 during the whole course. All animals (n=9) in the blank control group had mucositis score > 3.0 at peak (Day 18), whereas 6 of them had score >= 4.0. All animals (n=9) in the vehicle group had mucositis score > 3.0 at peak (Day 18), whereas 5 of them had score >= 4.0. The mean peak mucositis score was 2.3 in the ASN-02 group compared with 3.75 in the vehicle and blank control groups. In addition to preventing moderate and severe mucositis, ASN-02 improved mild mucositis at the peak to normal appearance in 2 days. The loss of body weight, activity, and appetite correlated well with the severity of mucositis in each group. Taken together, the ASN-02 (phenylbutyrate) oral gel reduced the mean clinical mucositis scores relative to the vehicle and blank controls in incidence, severity and duration of radiation-induced oral mucositis, and prevented the clinical manifestations of fatigue syndrome and malnutrition.

### Example 3: hong-term Suppression of the Radiation-induced Aberrant Proinflammatory Cytokine by an HDAC Inhibitor

Development of radiation-induced mucositis has been attributed to the radiation-induced persistent up-regulation of proinflammatory cytokines such as TNF-α. Levels of mRNA of the major proinflammatory cytokine, TNF-α, were assessed using a multiple cytokine RNase protection assay kit (Riboquant; Pharmingen, San Diego, CA) that contained a template set to allow for the generation of a 32P-labeled antisense RNA probe set that hybridized with the target TNF-α mRNA and the internal control GAPDH. After hybridization of labeled probe to target RNA, unprotected RNA was digested by a ribonuclease (RNase), and protected RNA fragments were resolved on a 6% polyacrylamide gel and recorded by phosphorimaging (Molecular Dynamics Corp., Sunnyvale, CA). Densitometry was used to quantify the amount of each mRNA species and was normalized to the internal control GAPDH. The irradiated mucosa (left buccal) and the nonirradiated mucosa (right buccal) were removed for assays at the same time as indicated.

The timing of the peak appearance of TNF-α upregulation induced by radiation correlated with the development of mucositis in all groups as shown in FIG. 3. In the ASN-02 (phenylbutyrate) group, the highest surge of TNF-α appeared at 1 day after irradiation, but levels were subsequently suppressed after Day 14. The suppression still persisted at 12 months. In the blank and vehicle control groups, mRNA levels of TNF-α in the irradiated mucosa increased and fluctuated above the nonirradiated mucosal levels over a period of 1 year and reached the first peak of 2-3-fold above the nonirradiated mucosal levels at 1 day after irradiation, the second peak of 10.5-16-fold around 14-28 days after irradiation, and the third peak of 13-14-fold at 9 months after irradiation; levels then declined to 2-3-fold normal levels by 12 months after irradiation.

This result indicates that the HDAC inhibitor can suppress the long-term upregulation of TNF-α to decrease the acute and chronic side effects induced by radiation.

### OTHER EMBODIMENTS

From the above description, one skilled in the art can easily ascertain the essential characteristics of the invention, and without departing from the spirit and scope thereof, can make various changes and modifications of the invention to adapt it to various usages and conditions. For example, compounds structurally and functionally analogous to HDAC inhibitors described above can also be used to practice the invention. Thus, other embodiments are also within the claims.

## Claims

1. Use of a pharmaceutical composition for the manufacture of a medicament for treating and/or preventing acute and chronic gastrointestinal distress including nausea, vomiting, lactose intolerance, obstructive symptoms, diarrhea, mucositis, bleeding, weight loss, or malnutrition, wherein the pharmaceutical composition comprises a histone deacetylase (HDAC) inhibitor or a pharmaceutically acceptable salt thereof, and a pharmaceutically acceptable carrier.

2. Use as claimed in claim 1, wherein the pharmaceutical composition is administered to the oral, pharyngeal, esophageal, or gastrointestinal tissues of a subject who is immunocompromised or receives a planned course of chemotherapy and/or radiotherapy.

3. Use as claimed in claim 1, wherein the HDAC inhibitor is a hydroxamic acid derivative, a fatty acid, a cyclic tetrapeptide, a benzamide derivative, or an electrophilic ketone derivative.

4. Use as claimed in claim 3, wherein the hydroxamic acid derivative is selected from a group consisting of suberoylanilide hydroxamic acid (SAHA), pyroxamide, M-carboxycinnamic acid bishydroxamide (CBHA), trichostatin A (TSA), trichostatin C, salicylihydroxamic acid (SBHA), azelaic bishydroxamic acid (ABHA), azelaic-1-hydroxamate-9-anilide (AAHA), 6-(3-chlorophenylureido) carpoic hydroxamic acid (3C1-UCHA), oxamflatin, A-161906, scriptaid, PXD-101, LAQ-824, cyclic hydroxamic acid-containing peptide(CHAP), MW2796, and MW2996.

5. Use as claimed in claim 3, wherein the cyclic tetrapeptide is selected from a group consisting of trapoxin A, FR901228 (FK 228 or Depsipeptide), FR225497, apicidin, CHAP, HC-toxin, WF27082, and chlamydocin.

6. Use as claimed in claim 3, wherein the fatty acid is selected from a group consisting of sodium butyrate, isovalerate, valerate, 4-phenylbutyrate (4-PBA), 4-phenylbutyrate sodium (PBS), arginine butyrate, propionate, butyramide, isobutyramide, phenylacetate, 3-bromopropionate, tributyrin, valproic acid, and valproate.

7. Use as claimed in claim 3, wherein the benzamide derivative is selected from a group consisting of CI-994, MS-27-275 (MS-275), and a 3'-amino derivative of MS-27-275.

8. Use as claimed in claim 3, wherein the electrophilic ketone derivative is selected from a group consisting of a trifluoromethyl ketone and an α-keto amide.

9. Use as claimed in claim 1, wherein the HDAC inhibitor is Depudecin.

10. Use as claimed in claim 1, wherein the pharmaceutically acceptable carrier is selected from a group consisting of a biocompatible polymer having reverse-thermal gelation property, a polymer resin, a viscous polymer gel, a hydrogel, and a bioadhesive substance.

11. Use as claimed in claim 1, wherein the pharmaceutical composition further comprises a second agent selected from a group consisting of a second HDAC inhibitor, a 5-hydroxytryptamine3 (5-H₃) receptor antagonist, a dopamine receptor antagonist, a DOPA-5-HT₃ receptor antagonist, a neurokinin (NK)-1 receptor antagonist, an anti-histamine, an anticholinergics a non-steroid anti-inflammation drug, a steroid, a growth factor, a cytokine, an anti-oxidant agent, a tricyclic antidepressant, a sedative agent, cannabinoids, a vitamin, and an antibiotics.

12. Use as claimed in claim 11, wherein the second agent is administered concurrently.

13. Use as claimed in claim 11, wherein the second agent is administered sequentially.

14. Use as claimed in claim 11, wherein the second agent is administered in a different route of the first HDAC inhibitor.

15. Use as claimed in claim 11, wherein the dopamine receptor antagonist is phenothiazines, or butyrophenones.

16. Use as claimed in claim 11, wherein the 5-HT₃ receptor antagonist is selected from a group consisting of dolasetron, granisetron, ondansetron, palonosetron, and tropistron.

17. Use as claimed in claim 11, wherein the DOPA-5-HT₃ receptor antagonist is metoclopramide.

18. Use as claimed in claim 11, wherein the NK-1 receptor antagonist is selected from a group consisting of vofopitant, CP-122,721, CJ-11,794, L-758,298, and aprepitant.

19. Use as claimed in claim 11, wherein the antibiotic is selected from a group consisting of ganciclovir, acyclovir, famciclovir, and tetracycline.

20. Use as claimed in claim 11, wherein the growth factor is keratinocyte growth factor (KGF), or granulocyte macrophage-colony stimulating factor (GM-CSF).

21. Use as claimed in claim 11, wherein the anti-oxidant is selected from a group consisting of amifostine, benzydamine, and N-acetylcysteine.

22. Use as claimed in claim 11, wherein the vitamin is selected from a group consisting of nicotinamide, vitamin B complex, vitamin C, and vitamin E.

23. Use of a pharmaceutical composition for the manufacture of a medicament for treating and/or preventing cachexia, cancer-related fatigue or chronic fatigue syndrome, wherein the pharmaceutical composition comprises a histone deacetylase inhibitor or a pharmaceutically acceptable salt thereof, and a pharmaceutically acceptable carrier.

24. Use as claimed in claim 23, wherein the pharmaceutical composition is administered to the oral, pharyngeal, esophogeal, or gastrointestinal tissues of a subject who is immunocompromised or receives a planned course of chemotherapy and/or radiotherapy.
